# EUROPEAN PATENT APPLICATION

(11) **EP 0 985 418 A2**
(43) Date of publication of application: **15.03.2000**
(21) Application number: 99202975.1
(22) Date of filing: 13.09.1999
(51) Int. Cl.: A61M 5/142

(54) **Device for the automatic injection of liquids**

(30) Priority: 15.09.1998 ES 9801935
(71) Applicant: Fernandez-Arias Montoya, Alberto, 50008 Zaragoza (ES)
(72) Inventor: Fernandez-Arias Montoya, Alberto, 50008 Zaragoza (ES)
(74) Representative: Ungria Lopez, Javier

(57) **Abstract**

Device for the automatic injection of liquids, of utility for injecting any type of substance in a continuous manner or for artificial insemination and able to be used for animals or humans, the device (1) consisting of a tubular syringe body (2), which at one end narrows to a neck (3) to which an injection set is coupled, whilst at its other end it has a threaded part (8), incorporating on its inside a piston (4) which defines two mutually water-tight chambers (5) and (6), being closed hermetically at its threaded end by means of a threaded cap (9).

## Description

### OBJECT OF THE INVENTION

The present invention, according to the title of the present specification, consists of a device for the automatic injection of liquids, which is intended preferentially for the controlled injection of medication, and for artificial insemination.

The device is based on a tubular body similar to a syringe, in the interior of which has a piston which defines two mutually water-tight chambers, one of them being for the liquid to be injected and the other for holding elements which in their chemical reaction release hydrogen, which on expanding inside said chamber pushes the piston towards the opposite extremity, producing the expulsion of the liquid to be injected.

The continuous and controlled generation of hydrogen, causes the liquid to be injected in a constant form, optimising the therapeutic potential of the latter, so that the patient, animal or human, transports in its or his own body the syringe, given its reduced size, permitting a normal life to be led.

This manner of applying the liquid to be injected permits:
- labour costs to be reduced;
- less stress to be produced in the animals;
- injecting to be performed over a determined period;
- constant levels of the products administered to be maintained.

In addition, in the case of animals, this form of injecting products over a series of determined days, permits substances to be administered, the use of which in slow-release or depot form or by way of subcutaneous implants, are expressly prohibited by current legislation with respect to determined animals for consumption.

On the other hand, in the case of its employment for artificial insemination, the continuous depositing of semen, will cause viable spermatozoids to be present in the "fertilisation zone", at the moment in which the female ovulates thereby improving the fertilisation.

### FIELD OF APPLICATION.

The device for the automatic injection of liquids is of particular application for use in those patients (animals or humans) requiring the injection of any kind of liquid medication or therapeutic substance, over a determined period of time or in artificial insemination.

Thus, the device is placed on the animal, its hydrogen release reaction rate being adjusted in order that it injects the liquid it contains inside at the desired rate.

### BACKGROUND TO THE INVENTION.

Conventionally, the administration of medication by parenteral via is done by means of injection, drip-feeds, and subcutaneous implants.

Where animals are involved and they have to be administered injections, the inconvenience occurs when capturing the animal as many times as it has to be injected. This involves labour costs as well as anxiety for the animal in question, with the additional aggravation that should a dose not be administered for any of the different reasons that may arise, such as absence, illness, etc., the effect produced by the therapeutic treatment is diminished.

In addition, on occasions, with repeated injections, constant levels of the injected substances are not achieved, there being a peak in concentration after the injection which gradually decreases as the medicine metabolises or is excreted.

Some medicines have been formulated in slow-absorption or depot form in order to overcome this problem. The drawback is that not all medicines are available in the slow-absorption (depot) form, and that the slow-release form is prohibited for certain types of animals for consumption.

The second method cited (subcutaneous implants) has the disadvantage of being employable only with a very limited number of substances and its use is likewise prohibited for certain animals for consumption.

On the other hand, there exist electronic injectors which can move the piston of a standard syringe at a determined speed. These electronic injectors are inconvenient in that they are fragile and their unit price is extremely high, meaning their use is in practice prohibitive. Their size together with the noise they make have also been disadvantages raised by persons who have employed them.

Finally, the injection of medicines by drip-feed has the drawback that the patient must remain still while receiving the therapeutic solution and that the latter has to be of considerable volume to permit injection to take place over a period of many hours, given that it is operated merely by gravitational effect, and not by a driving mechanism.

In brief, at the present time there is no device which permits the injection of substances in the therapeutic concentrations normally employed, over a number of hours or days in a constant manner, which is at the same time robust and economical and which permits the patient to lead a normal life.

Likewise, in the case of artificial insemination, the drawback occurs through the semen being introduced in a single dose, so that if the ovulation time has not been well calculated, said dose will not achieve the desired effect, with the additional aggravation of not knowing at the time whether the insemination has produced the desired effect, and if, as a precaution, it has to be repeated, said insemination results in a stressful situation which can prove counterproductive regarding the desired conception.

### DESCRIPTION OF THE INVENTION.

In the present specification a device is described for the automatic injection of liquids, of utility for injecting any type of liquid over a period of time or for artificial insemination, the device consisting of a tubular syringe body, which at one extremity narrows to form a neck to which is coupled an injection set, while at the other extremity it is threaded, incorporating on the inside a piston which defines two mutually water-tight chambers, and at its threaded end it is hermetically sealed by a cap which is screwed on to it or other type of hermetic seal.

In the injection chamber defined in the tubular syringe body by the piston with respect to the extremity which has the neck for coupling with the injection set, the substance to be injected is deposited and in the second reaction chamber a metallic element is placed together with an acid, the tubular syringe body being closed by means of the threaded cap with watertight seal.

The metal deposited in the reaction chamber of the tubular syringe body together with the acid is defined by a metallic body variable in volume in accordance with the time desired for the automatic injection of the liquid to be injected to last.

Likewise, the solid metal body may have its respective ends fitted with fixtures of plastic, silicone or rubber.

Similarly, the metal deposited in the reaction chamber of the tubular syringe body together with the acid, can be in the form of metal shards or dust in variable quantity according to the time desired for the automatic injection of the liquid to be injected to last.

In this manner, when the acid reacts with the metal, hydrogen is produced which, on expanding, causes the slow and continuous advance of the piston into the injection chamber, producing the injection by parenteral via of the liquid contained in the injection chamber during the pre-established time, in accordance with the rate of reaction of the metal with the acid, which is intimately related with the rate of displacement of the piston.

The tubular syringe body during the injection time of the substance to be injected remains attached to the body of the patient to be injected, normally homothermous, protected from impact, maintaining a constant temperature and therefore having a constant rate of reaction of acid with metal, whereby the tubular syringe body causes no inconvenience for the patient, the latter being able to lead a normal life and without suffering any sensation of anxiety.

In this manner a saving in labour costs is achieved, since in the case of animals there is no requirement to capture them whenever they have to be injected, if the therapeutic treatment is carried out by periodic doses, the medication, moreover, having a greater therapeutic potential through the elimination of the peaks and valleys that occur after a standard injection.

When using the injector device for artificial insemination, the cannula of the injection set is positioned in the proximity of the "os cervix" of the uterus, slowly and gradually depositing the semen at said location, causing the spermatozoids to reach the "fertilisation zone" sequentially and there being viable spermatozoids available when the female ovulates, improving thereby the fertilisation process.

In order to complete the description to be given hereafter, and for the purpose of facilitating a better understanding of the characteristics of the invention, attached to this specification is a drawing containing a single figure which shows, by way of illustration and in no way limiting, the most characteristic details of the invention.

### BRIEF DESCRIPTION OF THE DESIGNS.

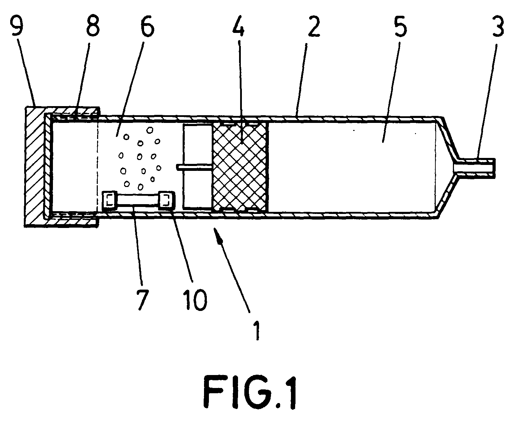

**Figure 1.-** Shows a view of a longitudinal diametrical cross section of the tubular syringe body, wherein can be seen the piston which defines the injection chamber in which is deposited the liquid to be injected and the reaction chamber in which the metallic element introduced reacts with the acid in order to generate hydrogen which on expanding produces the slow and continuous advance of the piston to achieve the injection of the pertinent liquid.

### DESCRIPTION OF A PREFERRED EMBODIMENT.

In the light of the figure mentioned and in accordance with the numbering adopted, it can be seen how the device 1 for the automatic injection of liquids, is essentially based on a syringe body 2 for the automatic injection of liquids or substances, being designed mainly for administering by parenteral via distinct substances or liquids to animals of different species, and even to humans under determined circumstances.

Thus, the device 1 consists of a tubular syringe body 2 provided with a piston 4 on its inside which defines an injection fore-chamber 5, with respect to its extremity provided with a neck 3, whilst regarding the rear part the piston 4 defines a reaction chamber 6, the tubular body 2 having its extremity relative to the reaction chamber 6 provided with a threaded part 8.

In this manner, onto the threaded extremity 8 of the tubular syringe body 2 is screwed a hermetically sealing cap 9 so that in the reaction chamber 6, a metallic element is deposited with an acid, in order that on reacting hydrogen is produced which in its slow and gradual expansion by occupying a greater volume, causes the piston 4 to advance along the injection chamber, producing the injection of the liquid contained in said injection chamber 5, by having coupled to the narrowed front extremity the pertinent injection set.

Thus, to the front extremity 3 of the syringe body 2, a "butterfly" or intravenous injection set has been coupled, such as the "Valu-set" type or any other commercially available make, so that once inserted in the spot where it is desired to inject the liquid, it is held in position by means of simple suture stitches, by means of powerful adhesive, or by other means.

The injector device is kept in intimate contact with the body of the animal by means of straps or collars, being protected on the side away from that in contact with the body of the animal from the outside temperature by means of an insulating material, whereby the body heat of the animal maintains the device at a constant temperature (as long as the animal in question is homothermous) whereby the reaction rate of the acid with the metal is also kept constant meaning that the displacement of the piston and the rate of injection are also constant. The injector is protected from possible impact by means of a rigid protective material.

To vary the rate of injection it is sufficient to vary the rate at which hydrogen is produced, which is achieved by means of changes in the reaction of the acid with the metal.

In this manner the metallic element 7 can have a volume that is variable according to the speed at which it is desired to have the piston 4 advance, it being possible to protect the sides of metallic element 7 by the pertinent body 10 of plastic or similar material.

Likewise, the metallic element introduced into the reaction chamber 6 in order that by reacting with the acid hydrogen is produced, and the consequent displacement of the piston 4, can be achieved materially by means of metal powder or shards, logically obtaining the same result.

Logically, device 1 offers an accessory to permit the insertion of the liquid to be injected, for which said accessory can be coupled to the piston via the base of the reaction chamber or else the introduction of the liquid to be injected can be performed by means of a conventional syringe through the neck 3.

The animal can therefore have the device properly attached to its own body, leading a normal life, with the added benefits of:
- Reducing labour costs, since it is not necessary to handle the animal each time it has to receive an injection, when the treatment is by a number of injections in series.
- Producing less anxiety in the animals, since by handling then fewer times, the resulting anxiety is diminished.
- Inject therapeutic liquids or substances over a determined number of days, when the use of said liquids or substances is not possible in slow-release form or subcutaneous implants.
- Maintain constant levels in the medication administered, optimising therapeutic potential, on being injected in a continuous manner.

In addition, in the use of the device for artificial insemination, the continuous depositing of semen means that viable spermatozoids are to be found on the "fertilisation site" at the moment in which the female ovulates, thereby improving the fertilisation process, since the cannula of the injection set has been situated close to the "os cervix" of the uterus, similarly eliminating the anxiety produced in the female through manipulation, which can affect the fertility.

## Claims

1. **DEVICE FOR THE AUTOMATIC INJECTION OF LIQUIDS,** of utility for injecting any type of substance in a continuous manner or for artificial insemination and able to be used with animals or humans, characterised in that the device (1) consists of a tubular syringe body (2), which at one extremity narrows to a neck (3) to which is coupled an injection set, whilst at the other extremity it has a threaded part (8), incorporating on the inside a piston (4) which defines two mutually watertight chambers (5) and (6), being hermetically closed at the threaded end by a screw cap (9).

2. **DEVICE FOR THE AUTOMATIC INJECTION OF LIQUIDS,** according to claim 1, characterised in that in the chamber (5) defined in the tubular syringe body (2) by the piston (4) with respect to the extremity which has the neck (3) the liquid to be injected is introduced, and in the second chamber (6), reaction chamber, a metallic element is deposited with an acid.

3. **DEVICE FOR THE AUTOMATIC INJECTION OF LIQUIDS,** according to claim 2, characterised in that the metallic element deposited in the reaction chamber (6) of the tubular syringe body (2) together with the acid, is defined by a metallic body (7) of variable area and volume.

4. **DEVICE FOR THE AUTOMATIC INJECTION OF LIQUIDS,** according to claim 2, characterised in that the metallic element deposited in the reaction chamber (6) of the tubular syringe body (2) together with the acid, is defined by a metallic body (7) of variable area and volume, which is held in position between respective bodies (10) of plastic, silicone or rubber.

5. **DEVICE FOR THE AUTOMATIC INJECTION OF LIQUIDS,** according to claim 2, characterised in that the metallic element deposited in the reaction chamber (6) of the tubular syringe body (2) together with the acid, is defined by metal powder or swarfs in variable amount.

6. **DEVICE FOR THE AUTOMATIC INJECTION OF LIQUIDS,** according to previous claims, characterised in that when the metal reacts with the acid, hydrogen is produced which on its expansion produces the slow and continuous displacement of the piston (4) in the injection chamber (5), injecting the liquid contained in the injection chamber (5) during the pre-established time.

7. **DEVICE FOR THE AUTOMATIC INJECTION OF LIQUIDS,** according to claim 1, characterised in that the tubular syringe body (2), during the time of injection, remains bound to the body of the homothermous patient to be injected, protected from impact, maintaining a constant temperature, and having a constant rate of reaction of the acid with the metal.

8. **DEVICE FOR THE AUTOMATIC INJECTION OF LIQUIDS,** according to claim 1, characterised in that in the use of the injector device (1) for artificial insemination, the cannula of the injection set, is positioned close to the "os cervix" of the uterus, depositing there the semen in a slow and gradual manner.
